# FASCICULE DE BREVET EUROPEEN

(11) **EP 2 598 412 B1**
(45) Date de publication et mention de la délivrance du brevet: **10.09.2014**
(21) Numéro de dépôt: 11755384.2
(22) Date de dépôt: 29.07.2011
(51) Int. Cl.: B65D 81/20, B65D 77/00, A61L 2/04, A61B 19/02

(54) **ARTICLE DE CONDITIONNEMENT JETABLE**
EINWEGSVERPACKUNG
DISPOSABLE PACKAGING

(30) Priorité: 30.07.2010 FR 1056346
(43) Date de publication de la demande: 05.06.2013
(73) Titulaire: ATMI BVBA, 3320 Hoegaarden (BE)
(72) Inventeur: ZAMBAUX, Jean-Pascal, 33980 Audenge (FR); MASSEAU, Daniel, 33000 Bordeaux (FR)
(74) Mandataire: Lienard, Benoît
(86) Numéro de dépôt international: PCT/FR2011/051841
(87) Numéro de publication internationale: WO 2012/013910

(56) Documents cités:
- WO-A1-2009/076409
- US-A- 5 806 283
- US-A1- 2004 146 669
- US-A1- 2009 100 802

## Description

L'invention concerne un article de conditionnement primaire ou secondaire jetable stérilisable et/ou dépyrogénable.

L'invention concerne en particulier un article de conditionnement de produits et/ou dispositifs du domaine médical, pharmaceutique, chimique, biologiques, alimentaire, ou électronique.

### ETAT DE L'ART

Dans le domaine de la stérilisation et/ou dépyrogénation par la chaleur de produits ou dispositifs conditionnés, ceux-ci sont mis dans l'enceinte d'un four avec un gradient de pression positive pour garder le produit et le conditionnement stérile et/ou dépyrogéné lors des phases de refroidissement. Le four doit donc être spécialisé pour ce type d'opération qui nécessite généralement une filtration stérilisante en continu de l'air à l'intérieur du four et un flux laminaire à la sortie de l'enceinte du four, notamment pour isoler l'intérieur de l'enceinte du four de l'atmosphère extérieure et garantir le maintien de la stérilisation en sortie.

Très récemment, la société DISPOSABLE-LAB (France) a inventé une nouvelle valve applicable aux articles de conditionnement de tels produits. Ce produit fait l'objet actuellement d'une demande de brevet internationale PCT/EP2010/053617. La présence de cette valve permet d'éviter d'utiliser un flux laminaire en sortie de l'enceinte de stérilisation et/ou dépyrogénation. Cependant le four doit être sous atmosphère stérile et en surpression lors de l'opération de stérilisation et/ou dépyrogénation.

### BUTS DE L'INVENTION

L'invention a pour but principal de résoudre le nouveau problème technique consistant en la fourniture d'un article de conditionnement utilisable dans tous types de four ou enceinte de stérilisation et/ou dépyrogénation. L'invention a donc pour but de permettre l'utilisation d'une enceinte de stérilisation et/ou dépyrogénation qui n'utilise pas de flux laminaire en sortie, ni d'atmosphère stérile en surpression qui ne sont pas des conditions d'utilisation avantageuses.

L'invention a également pour but de résoudre le problème technique particulier de la fourniture de flacons stériles et/ou dépyrogénés conditionnés dans un emballage stérile et/ou dépyrogéné.

L'invention a également pour but de résoudre le problème technique consistant en la fourniture d'un article de conditionnement permettant de garder le produit stérile et/ou dépyrogéné lors de sa conservation et jusqu'à son utilisation.

L'invention a également pour but de résoudre le problème technique consistant en la fourniture d'un procédé de stérilisation et/ou dépyrogénation d'un article de conditionnement jetable qui soit plus facilement réalisable et/ou à moindres coûts.

L'invention a également pour but de résoudre le problème technique consistant en la fourniture d'un procédé de stérilisation et/ou dépyrogénation d'un article de conditionnement jetable utilisable dans tout type d'appareillage de stérilisation ou dépyrogénation, notamment par une montée et maintien à une certaine température de stérilisation et/ou dépyrogénation.

L'invention a pour but de fournir une solution à l'ensemble des problèmes techniques mentionnés de manière simple, industrielle, et peu onéreuse. Notamment l'invention vise à fournir un article de conditionnement stérile facilement utilisable et jetable.

### DESCRIPTION DE L'INVENTION

L'invention concerne un article de conditionnement qui peut être stérilisé et/ou dépyrogéné, c'est-à-dire libéré des agents pyrogènes. Un agent pyrogène est un agent pouvant provoquer une fièvre chez un sujet humain ou animal. L'expression "libéré des agents pyrogènes" signifie que des agents pyrogènes ne sont pas présents ou en quantité inférieure aux standards acceptables pour l'utilisation du produit ou dispositif considéré. Le terme "dépyrogenation" se réfère en particulier au retrait des agents pyrogènes des dispositifs, ou produits, et plus particulièrement des produits pharmaceutiques injectables, des crèmes stériles, ou des dispositifs pharmaceutiques, médicaux ou chirurgicaux.

La présente invention couvre en particulier un article de conditionnement jetable (1) réalisé au moins en partie avec au moins un film d'un thermoplastique (10) résistant à une stérilisation et/ou dépyrogénation, et résistant à une température supérieure à 225°C à 10⁵ Pa, ledit article de conditionnement jetable (1) définissant au moins une enceinte intérieure (5) destinée à recevoir un ou plusieurs éléments (50) à conditionner, ladite enceinte intérieure (5) étant sous vide (40) et stérile et/ou dépyrogénée, ledit article de conditionnement (1) comprenant un ou plusieurs éléments (50) conditionnés.

Par « thermoplastique » il est fait référence à un matériau polymérique obtenu par polymérisation de monomères comprenant au moins un atome de carbone, et susceptible d'être, de manière répétée, ramolli par chauffage et durci par refroidissement.

Par « vide » on entend une dépression de l'enceinte intérieure par rapport à la pression extérieure à l'article de conditionnement dans des conditions de stockage. De préférence le vide est une pression comprise entre 1 et 500 hPa, de préférence entre 1 et 400 hPA, et par exemple d'environ 300 à 350 hPa.

Selon une variante, l'article de l'invention comprend au moins un orifice d'entrée et/ou de sortie d'un ou plusieurs éléments à conditionner dans l'enceinte intérieure de l'article, ledit au moins un orifice d'entrée et/ou de sortie étant fermé par au moins un moyen de fermeture après introduction des éléments à conditionner dans l'enceinte intérieure de l'article, comme par exemple au moyen d'au moins une soudure et/ou pince mécanique du film d'un thermoplastique résistant à une stérilisation et/ou dépyrogénation, et résistant à une température supérieure à 225 °C à 10⁵ Pa.

De préférence l'orifice d'entrée et/ou de sortie est formé par le film thermoplastique.

On entend par matérieu polymérique résistant à une stérilisation et/ou dépyrogénation, un matériau qui ne se dégrade pas ou sensiblement pas lors de la stérilisation et/ou dépyrogénation. Ce matériau ne doit notamment pas se retrouver en partie à l'état liquide ou gazeux, ni faire l'objet d'une dégradation préjudiciable à la forme de l'article et/ou aux produits ou dispositifs à conditionner. Typiquement la température de fusion de ce matériau est supérieure à 225 °C à 10⁵ Pa.

Avantageusement, l'enceinte intérieure de conditionnement est mise sous vide, de préférence avant ou simultanément à la fermeture par au moins un moyen de fermeture du ou des orifices d'introduction et/ou de sortie, comme par exemple une fermeture au moyen d'au moins une soudure ou action mécanique.

Selon un mode de réalisation, l'article de conditionnement jetable est une poche jetable, par exemple simple ou mutiple, réalisée d'un ou plusieurs films d'au moins un thermoplastique. Les bords extérieurs du ou des films de thermoplastique formant la périphérie de l'orifice d'introduction sont avantageusement soudés.

Selon un mode de réalisation l'article de conditionnement jetable présente des parois formées d'au moins un film d'un thermoplastique résistant à une stérilisation et/ou dépyrogénation, et résistant à une température supérieure à 225 °C à 10⁵ Pa.

Avantageusement, ledit article comprend au moins un orifice d'introduction d'un ou plusieurs éléments à conditionner dans l'enceinte intérieure de l'article, le ou les orifices d'introduction et/ou de sortie étant fermés par soudure ou par un dispositif de pince mécanique du film de thermoplastique.

On utilise de préférence un seul thermoplastique, mais l'invention couvre également l'utilisation de plusieurs matériaux polymériques afin d'associer leurs propriétés pour améliorer les propriétés de l'article de conditionnement. Parmi les polymères préférés on utilise un ou plusieurs polymères choisis parmi : PE (Polyethylene), PP (Polypropylene), PFA (Perfluoroalkoxy), polyaryletherketone, FEP ( fluoro ethylène propylène ), PEEK (Poly(ether ether ketone)) (PEEK MT ou ST), PAI (Polyamide imide), PI (Polyimide), PPSU (Polyphénylène sulfone), PES (Polyéther sulfone), PPP (Polyparaphénylène) , POM (Polyoxyméthylène), et les polymères analogues. Un polymère préféré est le PEEK.

Ainsi l'article de l'invention est réalisé au moins en partie, et de préférence en totalité, de préférence en PEEK ou PFA.

Selon un mode de réalisation avantageux, la pression de l'enceinte intérieure est comprise entre environ 1 et 500 hPa, de préférence entre 1 et 400 hPA, et par exemple d'environ 300 à 350 hPa. La mise sous vide est effectuée de manière à limiter au maximum la dilatation de l'enceinte intérieure de l'article de conditionnement. Selon un mode de réalisation particulier on laisse une présence de gaz (air, oxygène, azote, ou autre gaz inerte, ou nécessaire au produit ou dispositif conditionné) suffisante pour permettre la dilatation de l'enceinte intérieure afin que les produits ou dispositifs soient en présence du même gaz, et afin que la stérilisation et/ou dépyrogénation soit validée, au moins du point de vue de sa qualité.

Avantageusement, l'article de l'invention comprend, de préférence proche d'un orifice d'introduction, un moyen d'ouverture facile pour déchirer le film de thermoplastique. Typiquement, une encoche en « V » est réalisée.

Selon une variante, le moyen d'ouverture facilité est une encoche, par exemple en forme de « V », réalisée dans le film de thermoplastique. De préférence l'encoche est réalisée de manière directionnelle afin de déchirer facilement le film plastique si le thermoplastique oppose une résistance qui est principalement directionnelle.

Avantageusement, l'article de l'invention comprend en tant qu'élément conditionné sous vide, un ou plusieurs récipients pour un ou plusieurs produits, et notamment des produits chimiques, pharmaceutiques, biologiques, ou alimentaires, ou un ou plusieurs dispositifs médicaux, chirurgicaux, ou électroniques.

Selon un mode de réalisation, l'enceinte intérieure est stérile et/ou dépyrogénée avec un ou plusieurs produits et/ou dispositif conditionnés dans l'enceinte intérieure.

Selon une variante, l'article de conditionnement est constitué par au moins un film d'un thermoplastique résistant à une stérilisation et/ou dépyrogénation, et résistant à une température supérieure à 225 °C à 10⁵ Pa, et encore de préférence en PEEK ou PFA, ledit film thermoplastique étant de forme tubulaire et fermé à ses extrémités pour définir ladite enceinte intérieure.

Avantageusement, l'article comprend un ou plusieurs flacons ou vials contenant des produits chimiques, pharmaceutiques, ou biologiques, à stériliser et/ou dépyrogéner.

Selon un mode de réalisation de l'invention, on place ces flacons ou vials sur un plateau ou support comprenant des séparateurs de flacon ou vials. L'ensemble du plateau et des flacons ou vials est placé dans l'enceinte intérieure pour sa stérilisation et/ou dépyrogénation.

Selon un mode de réalisation préféré de l'invention, on dispose les flacons ou vials à l'aide d'un ou plusieurs éléments séparateurs de flacon ou vial. L'ensemble flacons/vials et élément(s) séparateur(s) est placé dans l'enceinte intérieure pour sa stérilisation et/ou dépyrogénation. Dans ce mode de réalisation on notera donc l'absence de tout support ou plateau dans l'article de conditionnement et la seule présence d'un ou plusieurs élément(s) séparateur(s) de flacon ou vial.

Avantageusement, l'élément séparateur est un élément comprenant des cloisons définissant des logements contigus permettant de recevoir, de préférence à intervalle régulier, les flacons, vials ou tout autre produit destinés à être conditionnés.

De préférence, sous forme conditionnée, l'élément séparateur est maintenu en place uniquement à l'aide de la pression réduite appliquée dans l'enceinte intérieure agissant par compression progressive du film thermoplastique. En d'autres termes, lorsque, par exemple, des flacons ou vials sont utilisés, l'élément séparateur peut être maintenu à une hauteur comprise entre la partie inférieure et la partie supérieure des flacons essentiellement grâce à la pression réduite (le vide) régnant dans l'enceinte intérieure. En pratique, la mise sous pression réduite entraine le déplacement progressif de l'élément séparateur d'une position « au repos » correspondant, de préférence, à la partie inférieure des flacons à une position « sous vide » correspondant, de préférence, à une position médiane située entre la partie inférieure et la partie supérieure des flacons.

Selon un autre mode de réalisation, l'invention ne comporte ni plateau ou support ni éléments séparateurs. En d'autre termes, l'enceinte intérieure de l'article de conditionnement selon l'invention ne contient que, par exemple, les produits, récipients, flacons ou vials destinés à être conditionnés.

Selon un mode de réalisation particulier, l'article de l'invention renferme dans l'enceinte intérieure de l'article de conditionnement un ou plusieurs articles de conditionnement jetable tel que défini précédemment, par exemple pour constituer au moins une double poche. Cette double poche, triple poche, ou multiple poche, permet de stériliser et/ou dépyrogéner l'extérieur de la première poche.

Selon un mode de réalisation préféré, des thermoplastiques différents sont utilisés pour réaliser les différents films d'une double poche, triple poche, ou multiple poche. Selon une variante, un thermoplastique différent est utilisé pour chaque couche. Avantageusement, la double poche, triple poche, ou multiple poche comprend un film de PEEK formant la couche la plus proche des éléments à conditionner, et un film en PFA pour la couche la plus éloignée formant la couche extérieure de l'article de conditionnement. Ceci permet de combiner différentes propriétés des thermoplastiques, et notamment d'éviter un phénomène de collage des films d'un même thermoplastique les uns aux autres lors d'une montée en température, comme lors d'une phase de stérilisation et/ou dépyrogénation.

Le film de thermoplastique de l'invention est généralement d'une épaisseur comprise entre 25 et 500 microns, de préférence entre 300 et 50 microns et souvent d'environ 200 microns.

Selon un mode de réalisation, l'article de conditionnement est une poche partiellement ou totalement flexible. La flexibilité peut être donnée notamment en faisant varier la nature du thermoplastique et/ou l'épaisseur du film. Il est également possible de prévoir qu'une partie de l'article de conditionnement comprend des parois latérales et un fond pour recevoir des produits, dispositifs, flacons ou vials, et que l'orifice d'entrée et/ou sortie (surface supérieure du pavé) est fermé par un film thermoplastique soudé à la périphérie des bords des parois latérales. Les parois latérales et le fond peuvent être en matériau rigide. Le film peut être retiré. Le fond de l'article peut constituer une surface de support des produits, dispositifs, flacons ou vials. Chaque produits, dispositifs, flacons ou vials peut être placé dans un compartiment individuel.

L'invention concerne ainsi un produit et/ou dispositif stérilisé et dépyrogéné sous vide partiel, prêt à l'emploi, contenu dans un article de conditionnement tel que défini ci-dessus et ci-après. Le produit et/ou dispositif conditionné de l'invention peut être ôté de son article de conditionnement par un simple geste de déchirement du film de thermoplastique, en particulier au niveau du ou des moyens d'ouverture facile.

L'invention concerne également un procédé de stérilisation et/ou dépyrogénation d'un article de conditionnement jetable, de préférence tel que défini précédemment, caractérisé en ce qu'il comprend :
- l'introduction d'un ou plusieurs produits et/ou dispositifs à l'intérieur de l'enceinte intérieure d'un article de conditionnement jetable réalisé au moins en partie avec un film d'un thermoplastique résistant à une stérilisation et/ou dépyrogénation, et résistant à une température supérieure à 225 °C à 10⁵ Pa, et de préférence dont les parois sont formées d'un tel film d'un thermoplastique résistant à une stérilisation et/ou dépyrogénation, et résistant à une température supérieure à 225 °C à 10⁵ Pa , ladite introduction étant réalisée au moyen d'au moins un orifice d'introduction défini dans ledit article ;
- la mise sous vide de l'article de conditionnement jetable ; et
- la fermeture, de préférence par soudure ou pince mécanique du film de thermoplastique, du ou des orifices d'introduction ; et
- la stérilisation et/ou dépyrogénation de l'article de conditionnement jetable refermant dans son enceinte intérieure mise sous vide un ou plusieurs produits et/ou dispositifs.

L'article de conditionnement contient ainsi un ou plusieurs produits et/ou dispositifs stérilisés et/ou dépyrogénés.

La Pharmacopée Européenne, 5.1.1 « méthodes de préparation des produits stériles » défini des méthodes de stérilisation dont le stérilisation par la chaleur sèche.

La mise sous vide de l'enceinte interne peut être effectuée par l'intermédiaire d'un système d'aspiration du gaz de l'enceinte interne. Le système d'aspiration peut être connecté à un tube débouchant dans l'enceinte interne. Une fois le vide réalisé, le tube est retiré en operculant l'espace laissé par le tube retiré puis avant le retrait total du tube, la fermeture est réalisée pour rendre imperméable l'enceinte interne.

L'étape de fermeture comprend selon une variante un soudage du film de thermoplastique. Un soudage typique réalise une double soudure linéaire et continue imperméabilisant l'enceinte interne de l'atmosphère extérieure à l'article de conditionnement.

Avantageusement, le procédé comprend l'introduction dans une enceinte fermée, comme par exemple un four, de l'article de conditionnement jetable mis sous vide, puis la stérilisation et/ou dépyrogénation de l'article de conditionnement jetable, puis le retrait de l'article de conditionnement jetable de l'enceinte fermée.

La soudure du film thermoplastique est réalisée notamment par une technique d'assemblage permanent qui assure une continuité de même nature par fusion des surfaces à joindre, rapprochement, contact puis maintien jusqu'à solidification du mélange ainsi formé. Le soudage peut être réalisé en particulier à l'air chaud, par friction, au miroir, par ultrasons, à hautes fréquences, ou par laser.

D'autres buts, caractéristiques et avantages de l'invention apparaîtront plus clairement à la lecture de la description explicative faite en référence aux modes de réalisation illustrés par les figures annexées dans lesquelles :

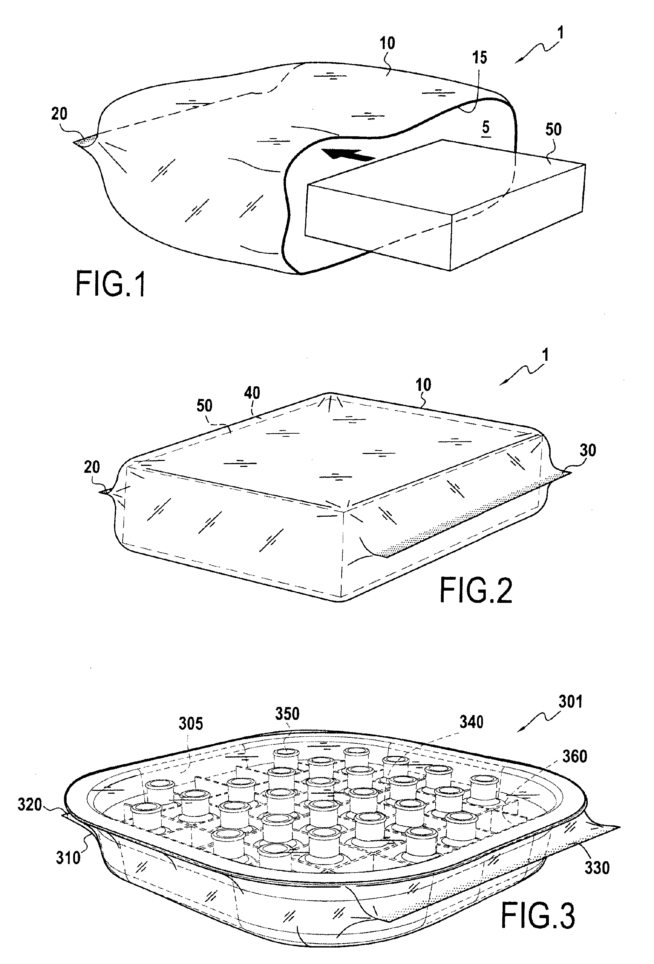

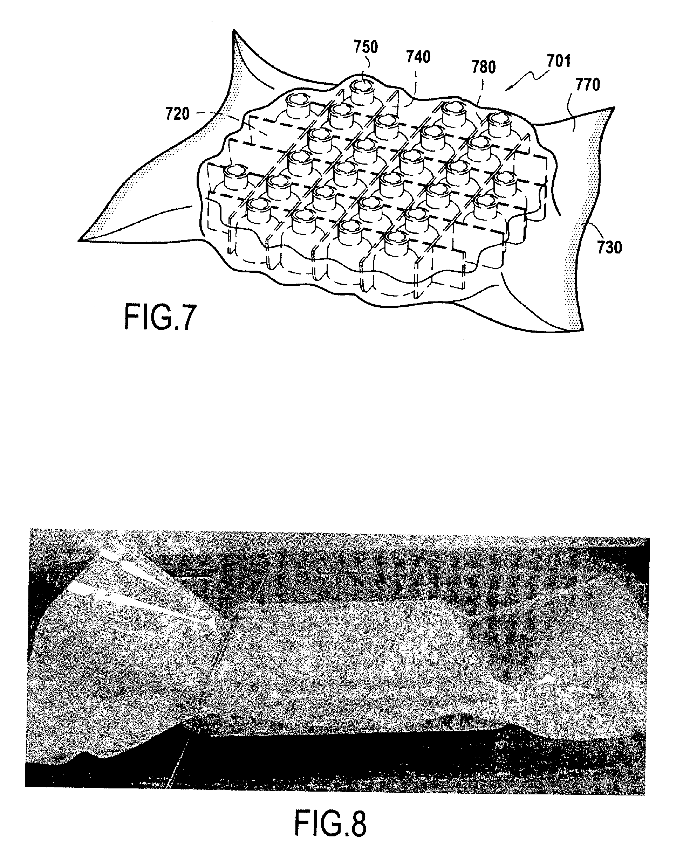

- La figure 1 illustre un mode de réalisation dans lequel un article de conditionnement jetable (1) est réalisé au moins en partie, et ici en totalité, avec un film d'un thermoplastique (10) résistant à une stérilisation et/ou dépyrogénation, et résistant à une température supérieure à 225 °C à 10⁵ Pa. Ce film est en PEEK. Ledit article de conditionnement jetable (1) défini au moins une enceinte intérieure (5) destinée à recevoir un ou plusieurs éléments (50) à conditionner. Le film (10) comprend une première soudure (20) fermant une extrémité de l'article de conditionnement. Cette soudure est également visible sur les modes de réalisation des figures 2 et 3 -20, 320).
- Les figures 2 et 3 illustrent un article de conditionnement jetable (1, 301) selon le mode selon le mode de réalisation de la figure 1 dans lequel l'enceinte intérieure (5, 305) de l'article (1, 301) est sous vide (40, 340) et stérile et/ou dépyrogénée, ledit article de conditionnement (1, 301) comprenant un ou plusieurs éléments (50, 350) conditionnés. Les éléments (50, 350) conditionnés sont stériles et/ou dépyrogénés. Ceci est obtenu après introduction des éléments (50, 350) à conditionner puis fermeture du film thermoplastique (10, 310), ici par soudure (30, 330) bord à bord au niveau de l'orifice d'introduction (15, 315). L'extraction d'un ou plusieurs éléments (50, 350) conditionnés peut se réaliser par l'orifice d'introduction (15, 315) qui sert alors d'orifice de sortie, ou par déchirement du film (10, 310) à un autre endroit quelconque ou prédéfini, typiquement par une encoche facilitant le déchirement du film (10).
- La figure 3 illustre un article de conditionnement jetable (301) dans lequel les éléments (350) conditionnés sont sous vide (340), stériles et/ou dépyrogénés dans l'enceinte intérieure (305) et placés sur un plateau comprenant des séparations (360). Ces éléments (350) sont ici des flacons qui peuvent contenir un produit d'intérêt en chimie, pharmacie, biologie, biotechnologie, ou alimentaire, ;

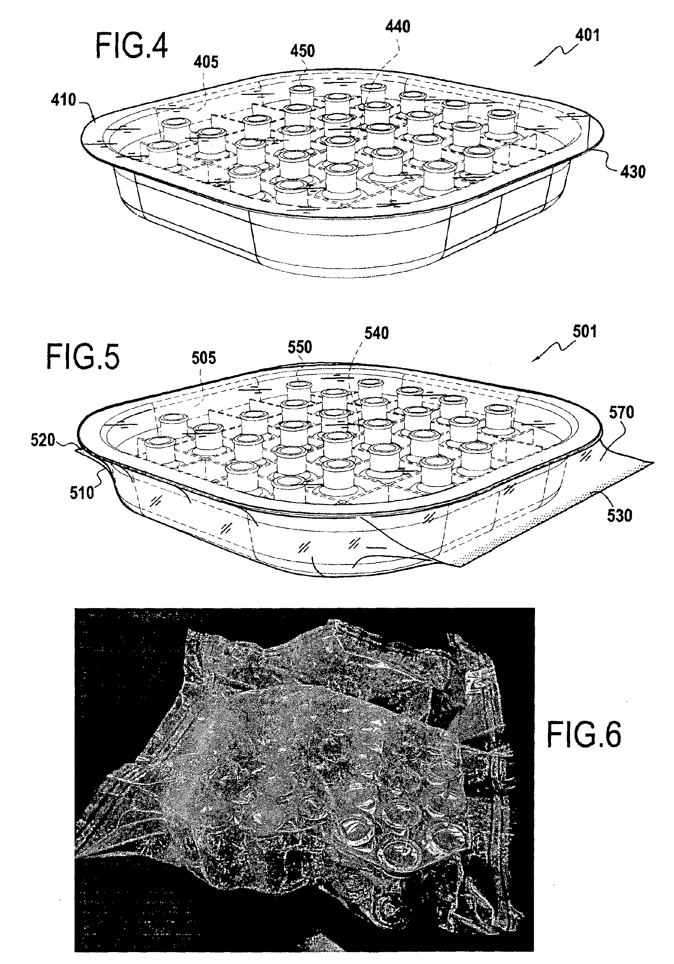

- La figure 4 illustre un mode de réalisation de l'invention dans lequel un article de conditionnement jetable (401) est réalisé au moins en partie avec un film d'un thermoplastique (410) résistant à une stérilisation et/ou dépyrogénation, et résistant à une température supérieure à 225 °C à 10⁵ Pa. Ce film est en PEEK. Ledit article de conditionnement jetable (401) défini au moins une enceinte intérieure (405) destinée à recevoir un ou plusieurs éléments (450) à conditionner. Le film (410) est soudé par une soudure (430) solidarisant le film (410) à la périphérie de la surface supérieure de l'enceinte intérieure (405) de l'article de conditionnement (401). Les éléments (450 sont conditionnés sous vide (440).

Dans un mode préféré de l'invention l'enceinte intérieur comprend un volume tampon ou volume « mort » permettant de limiter les forces exercées au niveau du moyen de fermeture lorsque le volume de l'enceinte intérieur augmente. Typiquement, lors de la procédure de stérilisation et/ou dépyrogénation, la montée en température implique une augmentation du volume de l'enceinte intérieure. Le volume tampon sert donc de zone de dilatation du gaz présent dans l'enceinte intérieure. Une zone tampon typique comprend une extension du film de thermoplastique non en regard des élément à conditionnés de plusieurs centimètre, et typiquement de 5 à 10 cm.
- La figure 5 représente une variante de réalisation du volume tampon (570). Les autres références sont déduites en ajoutant 500 aux références des figures 1 et 2.
- La figure 6 est une photographie d'un mode de réalisation de l'invention. Dans cette enceinte intérieure comprenant des flacons, le vide est d'environ 320hPa.
- La figure 7 illustre un article de conditionnement (1, 701) dans lequel les éléments (750) conditionnés sont sous vide (740), stériles et/ou dépyrogénés dans l'enceinte intérieure (720) et séparés par des éléments séparateurs (780). Les autres références sont déduites en ajoutant 700 aux références des figures 1 et 2.
- La figure 8 est une photographie d'un mode de réalisation de l'invention. Dans cette enceinte intérieure, un élément séparateur comprenant des cloisons définissant des logements contigus est utilisé pour assurer la séparation des flacons.

Les exemples qui viennent d'être donnés font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

## Revendications

1. Article de conditionnement jetable (1) réalisé au moins en partie avec au moins un film d'un thermoplastique (10) résistant à une stérilisation et/ou dépyrogénation et résistant à une température supérieure à 225 °C à 10⁵ Pa, ledit article de conditionnement jetable (1) définissant au moins une enceinte intérieure (5) destinée à recevoir un ou plusieurs éléments (50) à conditionner, ladite enceinte intérieure (5) étant sous vide (40) et stérile et/ou dépyrogénée, ledit article de conditionnement (1) comprenant un ou plusieurs éléments (50) conditionnés.

2. Article, selon la revendication 1, **caractérisé en ce que** l'article (1) comprend au moins un orifice (15) d'entrée et/ou de sortie d'un ou plusieurs éléments (50) à conditionner dans l'enceinte intérieure (5) de l'article (1), ledit au moins un orifice (15) d'entrée et/ou de sortie étant fermé par au moins un moyen de fermeture après introduction des éléments à conditionner dans l'enceinte intérieure de l'article, comme par exemple au moyen d'au moins une soudure et/ou pince mécanique du film d'un thermoplastique (10) résistant à une stérilisation et/ou dépyrogénation et résistant à une température supérieure à 225 °C à 10⁵ Pa.

3. Article, selon la revendication 1 ou 2, **caractérisé en ce que** l'enceinte intérieure (5) de conditionnement est mise sous vide, de préférence avant ou simultanément à la fermeture par au moins un moyen de fermeture (20, 30) du ou des orifices (15) d'introduction et/ou de sortie, comme par exemple une fermeture au moyen d'au moins une soudure ou action mécanique.

4. Article, selon l'une quelconque des revendications précédentes, **caractérisé en ce que** l'article de conditionnement jetable (1) présente des parois formées d'au moins un film d'un thermoplastique (10) résistant à une stérilisation et/ou dépyrogénation et résistant à une température supérieure à 225 °C à 10⁵ Pa.

5. Article, selon l'une quelconque des revendications précédentes, **caractérisé en ce que** ledit article (1) comprend au moins un orifice (15) d'introduction et/ou de sortie d'un ou plusieurs éléments (50) à conditionner dans l'enceinte intérieure (5) de l'article (1), le ou les orifices (15) d'introduction et/ou de sortie étant fermés par soudure ou un dispositif mécanique, comme un dispositif de pince mécanique du film de thermoplastique (10).

6. Article, selon l'une quelconque des revendications précédentes, **caractérisé en ce que** la pression de l'enceinte intérieure de l'article de conditionnement est comprise entre environ 1 et 500 hPA, de préférence entre 1 et 400 hPA, et par exemple d'environ 300 à 350 hPa.

7. Article, selon l'une quelconque des revendications précédentes, **caractérisé en ce qu'**il comprend, de préférence proche d'un orifice d'introduction, un moyen d'ouverture facile pour déchirer le film de thermoplastique.

8. Article, selon la revendication 7, **caractérisé en ce que** le moyen d'ouverture facile est une encoche en V réalisée dans le film de thermoplastique.

9. Article, selon l'une quelconque des revendications précédentes, **caractérisé en ce qu'**il comprend en tant qu'élément conditionné sous vide, un ou plusieurs récipients pour un ou plusieurs produits, et notamment des produits chimiques, pharmaceutiques, biologiques, ou alimentaires, ou un ou plusieurs dispositifs médicaux, chirurgicaux, ou électroniques.

10. **Article selon l'une quelconque des revendications précédentes, caractérisé en ce que ledit un ou plusieurs éléments conditionnés est un ou plusieurs flacons.**

11. Article, selon l'une quelconque des revendications précédentes, **caractérisé en ce qu'**il est réalisé au moins en partie, et de préférence en totalité, en PEEK ou PFA.

12. Article, selon l'une quelconque des revendications précédentes, **caractérisé en ce qu'**il est constitué par au moins un film d'un thermoplastique (10) résistant à une stérilisation et/ou dépyrogénation, et résistant à une température supérieure à 225 °C à 10⁵ Pa, de préférence en PEEK ou PFA, ledit film thermoplastique (10) étant de forme tubulaire et fermé à ses extrémités pour définir ladite enceinte intérieure.

13. Article, selon l'une quelconque des revendications précédentes, **caractérisé en ce qu'**il renferme dans l'enceinte intérieure de l'article de conditionnement un ou plusieurs articles définis selon l'une quelconque des revendications précédentes, par exemple pour constituer au moins une double poche.

14. Procédé de stérilisation et/ou dépyrogénation d'un article de conditionnement jetable, de préférence tel que défini selon l'une quelconque des revendications 1 à 13, **caractérisé en ce qu'**il comprend :
- l'introduction d'un ou plusieurs produits et/ou dispositifs (50) à l'intérieur de l'enceinte intérieure (5) d'un article de conditionnement jetable (1) réalisé au moins en partie avec un film d'un thermoplastique (10) résistant à une stérilisation et/ou dépyrogénation et résistant à une température supérieure à 225 °C à 10⁵ Pa, et de préférence dont les parois sont formées d'un tel film d'un, ladite introduction étant réalisée au moyen d'au moins un orifice d'introduction (15) défini dans ledit article (1) ;
- la mise sous vide de l'article de conditionnement jetable ;
- la fermeture, de préférence par soudure ou pince mécanique du film de thermoplastique (10), du ou des orifices d'introduction (15) ; et
- la stérilisation et/ou dépyrogénation, par une montée et maintien à une température de stérilisation et/ou dépyrogénation supérieure à 225 °C, de l'article de conditionnement jetable (1) refermant dans son enceinte intérieure (5) mise sous vide un ou plusieurs produits et/ou dispositifs (50).

15. Procédé, selon la revendication 14, **caractérisé en ce qu'**il comprend l'introduction dans une enceinte fermée, comme par exemple un four, de l'article de conditionnement jetable mis sous vide, puis la stérilisation ou dépyrogénation, par une montée et maintien à une température de stérilisation et/ou dépyrogénation supérieure à 225 °C, de l'article de conditionnement jetable, puis le retrait de l'article de conditionnement jetable de l'enceinte fermée.

## Patentansprüche

1. Einwegverpackungsartikel (1), der wenigstens teilweise mit wenigstens einer Folie aus einem Thermoplast (10), welcher einer Sterilisation und/oder Depyrogenisierung standhält und eine Temperatur von über 225 °C bei 10⁵ Pa verträgt, hergestellt ist, wobei der Einwegverpackungsartikel (1) wenigstens einen Innenraum (5) definiert, der dazu bestimmt ist, ein oder mehrere zu verpackende Elemente (50) aufzunehmen, wobei der Innenraum (5) unter Vakuum (40) steht und steril und/oder depyrogenisiert ist, wobei der Verpackungsartikel (1) ein oder mehrere verpackte Elemente (50) enthält.

2. Artikel nach Anspruch 1, **dadurch gekennzeichnet, dass** der Artikel (1) wenigstens eine Öffnung (15) zum Einführen und/oder Herausnehmen eines oder mehrerer in dem Innenraum (5) des Artikels (1) zu verpackender Elemente (50) umfasst, wobei die wenigstens eine Einführ- und/oder Herausnahmeöffnung (15) durch wenigstens ein Mittel zum Verschließen nach Einführen der in dem Innenraum des Artikels zu verpackenden Elemente verschlossen wird, wie zum Beispiel mittels wenigstens einer Schweißnaht und/oder eines mechanischen Zusammenkneifens der Folie aus einem Thermoplast (10), welcher einer Sterilisation und/oder Depyrogenisierung standhält und eine Temperatur von über 225 °C bei 10⁵ Pa verträgt.

3. Artikel nach Anspruch 1 oder 2, **dadurch gekennzeichnet, dass** der Verpackungsinnenraum (5) evakuiert wird, vorzugsweise vor oder gleichzeitig mit dem Verschließen durch wenigstens ein Mittel zum Verschließen (20, 30) der Einführ- und/oder Herausnahmeöffnung oder -öffnungen (15), wie zum Beispiel ein Verschließen mittels wenigstens einer Schweißnaht und/oder eines mechanischen Einwirkens.

4. Artikel nach einem der vorhergehenden Ansprüche, **dadurch gekennzeichnet, dass** der Einwegverpackungsartikel (1) Wände aufweist, die aus wenigstens einer Folie aus einem Thermoplast (10), welcher einer Sterilisation und/oder Depyrogenisierung standhält und eine Temperatur von über 225 °C bei 10⁵ Pa verträgt, ausgebildet sind.

5. Artikel nach einem der vorhergehenden Ansprüche, **dadurch gekennzeichnet, dass** der Artikel (1) wenigstens eine Öffnung (15) zum Einführen und/oder Herausnehmen eines oder mehrerer in dem Innenraum (5) des Artikels (1) zu verpackender Elemente (50) umfasst, wobei die Einführ- und/oder Herausnahmeöffnung oder -öffnungen (15) durch Verschweißen oder eine mechanische Vorrichtung, wie eine Vorrichtung zum mechanischen Zusammenkneifen der Thermoplastfolie (10) verschlossen werden.

6. Artikel nach einem der vorhergehenden Ansprüche, **dadurch gekennzeichnet, dass** der Druck des Innenraums des Verpackungsartikels zwischen etwa 1 und 500 hPa, vorzugsweise zwischen 1 und 400 hPa und beispielsweise etwa 300 bis 350 hPa beträgt.

7. Artikel nach einem der vorhergehenden Ansprüche, **dadurch gekennzeichnet, dass** er, vorzugsweise nahe einer Einführöffnung, ein Mittel zum leichten Öffnen umfasst, um die Thermoplastfolie aufzureißen.

8. Artikel nach Anspruch 7, **dadurch gekennzeichnet, dass** das Mittel zum leichten Öffnen eine in der Thermoplastfolie ausgebildete V-förmige Einkerbung ist.

9. Artikel nach einem der vorhergehenden Ansprüche, **dadurch gekennzeichnet, dass** er als vakuumverpacktes Element einen oder mehrere Behälter für ein oder mehrere Produkte, und insbesondere chemische, pharmazeutische, biologische Produkte oder Nahrungsmittel, oder eine oder mehrere medizinische, chirurgische oder elektronische Vorrichtungen enthält.

10. Artikel nach einem der vorhergehenden Ansprüche, **dadurch gekennzeichnet, dass** das eine oder mehrere verpackte Elemente ein oder mehrere Fläschchen ist.

11. Artikel nach einem der vorhergehenden Ansprüche, **dadurch gekennzeichnet, dass** er wenigstens teilweise, und vorzugsweise vollständig, aus PEEK oder PFA hergestellt ist.

12. Artikel nach einem der vorhergehenden Ansprüche, **dadurch gekennzeichnet, dass** er durch wenigstens eine Folie aus einem Thermoplast (10), welcher einer Sterilisation und/oder Depyrogenisierung standhält und eine Temperatur von über 225 °C bei 10⁵ Pa verträgt, vorzugsweise aus PEEK oder PFA, gebildet ist, wobei die Thermoplastfolie (10) röhrenförmig und an ihren Enden verschlossen ist, um den Innenraum zu definieren.

13. Artikel nach einem der vorhergehenden Ansprüche, **dadurch gekennzeichnet, dass** er in dem Innenraum des Verpackungsartikels einen oder mehrere nach einem der vorhergehenden Ansprüche definierten Artikel enthält, beispielsweise um wenigstens eine Doppeltasche zu bilden.

14. Verfahren zur Sterilisation und/oder Depyrogenisierung eines Einwegverpackungsartikels, vorzugsweise wie er nach einem der Ansprüche 1 bis 13 definiert ist, **dadurch gekennzeichnet, dass** es umfasst:
- das Einführen eines oder mehrerer Produkte und/oder Vorrichtungen (50) in den Innenraum (5) eines Einwegverpackungsartikels (1), der wenigstens teilweise mit einer Folie aus einem Thermoplast (10), welcher einer Sterilisation und/oder Depyrogenisierung standhält und eine Temperatur von über 225 °C bei 10⁵ Pa verträgt, hergestellt ist und dessen Wände vorzugsweise von einer solchen Folie eines gebildet sind, wobei das Einführen mittels wenigstens einer in dem Artikel (1) definierten Einführöffnung (15) vollzogen wird;
- das Evakuieren des Einwegverpackungsartikels;
- das Verschließen der Einführöffnung oder -öffnungen (15), vorzugsweise durch Verschweißen oder mechanisches Zusammenkneifen der Thermoplastfolie (10); und
- die Sterilisation und/oder Depyrogenisierung, durch einen Anstieg und Halten auf eine(r) Sterilisations- und/oder Depyrogenisierungstemperatur von über 225 °C, des Einwegverpackungsartikels (1), welcher in seinem evakuierten Innenraum (5) ein(e) oder mehrere Produkte und/oder Vorrichtungen (50) enthält.

15. Verfahren nach Anspruch 14, **dadurch gekennzeichnet, dass** es das Einführen in einen geschlossenen Raum, wie zum Beispiel einen Ofen, des evakuierten Einwegverpackungsartikels, dann die Sterilisation oder Depyrogenisierung des Einwegverpackungsartikels durch einen Anstieg und Halten auf eine(r) Sterilisations- und/oder Depyrogenisierungstemperatur von über 225 °C, anschließend das Herausnehmen des Einwegverpackungsartikels aus dem geschlossenen Raum umfasst.

## Claims

1. A disposable packaging article (1) made at least in part of at least one film of a thermoplastic (10) resistant to sterilisation and/or depyrogenation and withstanding a temperature higher than 225°C at 10⁵ Pa, the said disposable packaging article (1) defining at least one inner enclosure (5) intended to receive one or more elements (50) to be packaged, the said inner enclosure (5) being placed under a vacuum (40) and being sterile and/or depyrogenated, the said packaging article (1) comprising one or more packaged elements (50).

2. The article according to claim 1, **characterized in that** the article (1) comprises at least one insertion and/or removal orifice (15) for one or more elements (50) to be packaged inside the inner enclosure (5) of the article (1), the said at least one insertion and/or removal orifice (15) being sealed by at least one sealing means after inserting the elements to be packaged in the inner enclosure of the article, for example via welding and/or mechanical clamping of the film of a thermoplastic (10) resisting sterilisation and/or depyrogenation and withstanding a temperature higher than 225°C at 10⁵ Pa.

3. The article according to claim 1 or 2, **characterized in that** a vacuum is created in the inner packaging enclosure (5) preferably before or simultaneously with sealing by at least one sealing means (20, 30) of the insertion and/or removal orifice (s) (15), for example sealing by means of at least one weld or mechanical action.

4. The article according to any one of the preceding claims, **characterized in that** the disposable packaging article (1) has walls formed of at least one film of a thermoplastic (10) resistant to sterilisation and/or depyrogenation and withstanding a temperature higher than 225°C at 10⁵ Pa.

5. The article according to any one of the preceding claims, **characterized in that** the said article (1) comprises at least one insertion and/or removal orifice (15) for one or more elements (50) to be packaged inside the inner enclosure (5) of the article (1), the insertion and/or removal orifice(s) (15) being sealed by welding or a mechanical device such as a mechanical clamping device of the thermoplastic film (10).

6. The article according to any one of the preceding claims, **characterized in that** the pressure inside the inner enclosure of the packaging article is between about 1 and 500 hPA, preferably between 1 and 400 hPA for example between 300 and 350 hPA.

7. The article according to any one of the preceding claims, **characterized in that**, preferably close to an insertion orifice, it comprises easy opening means to tear the thermoplastic film.

8. The article according to claim 7, **characterized in that** the easy opening means are formed by a V-notch made in the thermoplastic film.

9. The article according to any one of the preceding claims, **characterized in that** as vacuum packed element it comprises one or more containers for one or more products, and in particular chemical, pharmaceutical, biological or food products, or one or more medical, surgical or electronic devices.

10. The article according to any one of the preceding claims, **characterized in that** the said one or more packaged elements are one or more bottles.

11. The article according to any one of the preceding claims, **characterized in that** it is made at least in part and preferably entirely in PEEK or PFA.

12. The article according to any one of the preceding claims, **characterized in that** it is formed by at least one film of a thermoplastic (10) resistant to sterilisation and/or depyrogenation and withstanding a temperature higher than 225°C at 10⁵ Pa, preferably in PEEK or PFA, the said thermoplastic film (10) being of tubular shape and sealed at its ends to define the said inner enclosure.

13. The article according to any one of the preceding claims, **characterized in that** inside the inner enclosure of the packaging article it encloses one or more articles defined according to any one of the preceding claims, for example to form at least a double pouch.

14. A method for sterilising and/or depyrogenating a disposable packaging article such as defined in any one of claims 1 to 13, **characterized in that** it comprises:
- inserting one or more products and/or devices (50) inside the inner enclosure (5) of a disposable packaging article (1) made at least in part with a film of a thermoplastic (10) resisting sterilisation and/or depyrogenation and withstanding a temperature higher than 225°C at 10⁵ Pa, the walls of which are preferably formed of a said film, the said inserting being performed via at least one insertion orifice (15) defined in the said article (1);
- creating a vacuum in the disposable packaging article;
- sealing the insertion orifice(s) (15), preferably by welding or mechanical clamping of the film of thermoplastic (10); and
- via a temperature rise and temperature hold at a sterilisation and/or depyrogenation temperature, sterilising and/or depyrogenating the disposable packaging article (1) enclosing in its vacuum sealed inner enclosure (5) one or more products and/or devices (50).

15. The method according to claim 14, **characterized in that** it comprises placing the vacuum sealed disposable packaging article in a closed chamber for example an oven, sterilising or depyrogenating the disposable packaging article by a temperature rise and temperature hold at a sterilisation and/or depyrogenation temperature higher than 225°C, then removing the disposable packaging article from the closed chamber.
